# EUROPEAN PATENT APPLICATION

(11) **EP 3 785 697 A1**
(43) Date of publication of application: **03.03.2021**
(21) Application number: 19194670.6
(22) Date of filing: 30.08.2019
(51) Int. Cl.: A61K 8/49, A61K 8/44, A61K 8/64, A61K 8/9789, A61Q 5/00, A61Q 11/00, A61K 31/4412, A61P 29/00, A61P 17/10

(54) **COSMETIC COMPOSITIONS COMPRISING PIPERLONGUMINE**

(71) Applicant: Unilever N.V., 3013 AL Rotterdam (NL)
(72) Inventor: CHEN, Yu, Shanghai, 200335 (CN); CUI, Xiao, Shanghai, 200335 (CN); HUANG, Dandan, Shanghai, 200335 (CN); HUANG, Nan, Shanghai, 200120 (CN)
(74) Representative: Tansley, Sally Elizabeth

(57) **Abstract**

Disclosed is a cosmetic composition comprising piperlongumine and at least one of a sulphur-containing amino acid comprising disulphide functional group or a peptide comprising disulphide functional group. Also disclosed is a method of widening the therapeutic window of piperlongumine for its use in a cosmetic composition for cosmetic treatment of an inflammatory condition including acne, dandruff and gingivitis, comprising including in said composition at least one of a sulphur-containing amino acid comprising disulphide functional group or a peptide comprising disulphide functional group.

## Description

### Field of the Invention

The present invention relates to a cosmetic composition for cosmetic treatment of inflammatory conditions such as gingivitis, acne and dandruff. Accordingly, the compositions of the invention could be formulated, for example, as a skincare, scalp care, haircare or an oral care composition.

### Background of the Invention

Inflammation is a biological response of a host to any harmful stimuli. It is a mechanism by which the host removes the stimuli to initiate the healing process for self-protection. The innate immune system of a host, such as a human being, acts as the first line of defence in a non-specific manner against invading organisms. Dysregulated inflammation may cause a variety of problems of cosmetic nature such as gingivitis (in the oral cavity), dandruff (on scalp/ hair) and acne on the skin. To assist the defence mechanism of the host organism (e.g., the human being), several anti-inflammatory agents have been used to mitigate the problems through topical application.

Some food preparations and beverages tend to have a negative impact oral hygiene. Acidic drinks and sweets, for example, can erode the teeth by attacking the enamel, which is the outer coating that protects our teeth. Food preparations which are sticky could accumulate in the interdental spaces and between the gums and the teeth. If the oral hygiene is not good enough, the particles of such food preparations and beverages tend to harden over time with help from the bacteria present in our mouth to form a film known as plaque.

Gingivitis is an inflammatory condition of the gums caused by accumulation of plaque. In gingivitis, the bacteria residing in the biofilms of the plaque and their corresponding components interact with the gingival tissues. As a result, the innate immune response gets activated, which is characterised by the release of pro-inflammatory cytokines. Gingivitis is a mild phase of periodontal disease and is defined as reversible inflammation. It is believed that good oral hygiene e.g., brushing and use of mouth wash products with therapeutic anti-microbial and anti-inflammatory efficacy, can be an effective way to reduce accumulated plaque that causes gingivitis and to alleviate the inflammatory response during gingivitis.

Dandruff affects many people. This condition varies from mild symptoms such as flaking skin, to severe inflammation and itchiness of the scalp. Malassezia yeasts, such as *Malassezia furfur*, are believed to be the main cause of dandruff and, whilst this might not represent the full scientific picture of the situation, Malassezia yeasts do appear to be closely associated with dandruff. Hence, conventionally dandruff is treated by topical application of antifungal agents such as zinc pyrithione (ZPTO), Octopirox®, climbazole and ketoconazole which are usually delivered through a shampoo. In addition, some anti-dandruff products also contain anti-inflammatory agents to alleviate the ill-effects of such conditions.

Acne affects a lot of people around the world. This condition has a displeasing cosmetic appearance. It has a complex etiology involving abnormal keratinization, excess production of sebum, excessive androgen function, excessive bacterial growth and immune hypersensitivity. Factors that have been linked to acne include the presence of free radicals with subsequent oxidative stress leading to cellular damage. It has been observed that acne usually occurs in areas rich in sebaceous glands like the face, neck and back. A bacterium, *Propionibacterium acnes* (*P*. *acnes*) is also a known causative factor.

The inflammatory conditions discussed earlier could manifest themselves in the form of a variety of cosmetic conditions such as acne, gingivitis and dandruff.

WO2014014515 A2 (Majeed Muhammed) discloses the use of piperlongumine to inhibit melanogenesis.

WO16014625 A1 (Bioventures LLC) discloses compositions and methods for selectively killing senescent cells that comprise piperlongumine or its derivative. The selective killing is used to delay aging or to treat age-related disorders.

In Cell Death and Disease (2013) 4, e824; doi:10.1038/cddis.2013.358, the authors, Wang et.al., have disclosed that N-acetyl-cysteine, an antioxidant, reduces piperlongumine-induced autophagy and cell death.

US2017/0246155 A1 (Bioventures LLC) discloses that N-acetyl-L-cysteine reduces cytotoxicity of piperlongumine.

In J Mol Med (2003) DOI: 10.1007/s00109-003-0422-2, the authors have disclosed that inhibition of proteasome is a strategy that can be used in anti-inflammation.

In Biochem J. (2013) 1;454(2):201-8. doi: 10.1042/BJ20130282, the authors showed that N-acetyl-L-cysteine could direct conjugation to piperlongumine, which leads to inhibition of piperlongumine's proteasome inhibition bioactivity.

The publications indicate that sulphur-containing amino acids such as N-acetyl-L-cysteine, may not expand or widen the anti-inflammatory therapeutic window of piperlongumine due to interference with its bioactivity.

In contrast to the findings reported in the publications, the present invention is based, in part, on a surprising finding that, e.g., L-cystine can reduce or at least partly nullify the cytotoxic effect of piperlongumine when the two are used in combination. Further L-cystine has been found to have little or practically no effect on the beneficial anti-inflammatory activity of piperlongumine, which thereby permits formulation scientists to formulate new and efficacious cosmetic compositions containing the two ingredients. Therefore, for example, L-cystine, which is a sulphur-containing amino acid having disulphide functional group, or a peptide having the same features could be used to expand or widen the anti-inflammation therapeutic window of piperlongumine.

### Summary of the Invention

Piperlongumine is a natural alkaloid obtained from fruits of long pepper (*Piper longum*) and other species of the genus Piper. *Piper longum* is a plant found in southern India and other Southeast Asian countries. Piperlongumine or long pepper oil (which contains piperlongumine) has been used in cosmetic compositions for its anti-inflammatory activity. However, the therapeutic window of piperlongumine is inherently narrow. Therapeutic window is a range of doses that produces the desired effect without causing any significant adverse effect to the user. In the context of cosmetic compositions, a wider window provides formulation flexibility to scientists to develop new products with the right balance of desired properties.

The present inventors have determined that when a sulphur-containing amino acid comprising disulphide functional group or a peptide comprising disulphide functional group is included in a cosmetic composition which comprises piperlongumine, the combination surprisingly widens the therapeutic window of piperlongumine in the treatment of cosmetic inflammatory conditions including acne, dandruff and gingivitis.

Such an observation can be utilised to formulate novel cosmetic compositions comprising piperlongumine and at least one of a sulphur-containing amino acid comprising disulphide functional group or a peptide comprising disulphide functional group.

Accordingly, in a first aspect disclosed is a cosmetic composition comprising piperlongumine and at least one of a sulphur-containing amino acid comprising disulphide functional group or a peptide comprising disulphide functional group.

In accordance with a second aspect is disclosed a method of widening the therapeutic window of piperlongumine for its use in a cosmetic composition for cosmetic treatment of an inflammatory condition including acne, dandruff and gingivitis, comprising including in said composition at least one of a sulphur-containing amino acid comprising disulphide functional group or a peptide comprising disulphide functional group.

In accordance with yet another aspect disclosed is use of a sulphur-containing amino acid comprising disulphide functional group or a peptide comprising disulphide functional group in a cosmetic composition comprising piperlongumine, to widen the therapeutic window of piperlongumine in said cosmetic composition for cosmetic treatment of an inflammatory condition including acne, dandruff and gingivitis.

In accordance with yet another aspect disclosed is a topical composition comprising piperlongumine and a sulphur-containing amino acid or protein comprising disulphide functional group for use in the treatment of inflammatory conditions including acne, dandruff and gingivitis.

In accordance with yet another aspect disclosed is use of a sulphur-containing amino acid comprising disulphide functional group or a peptide comprising disulphide functional group in the manufacture of a cosmetic composition comprising piperlongumine, to widen the therapeutic window of piperlongumine in said cosmetic composition for cosmetic treatment of an inflammatory condition including acne, dandruff and gingivitis.

### Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

Where a feature is disclosed with respect to a particular aspect of the invention (for example a composition of the invention), such disclosure is also to be considered to apply to any other aspect of the invention (for example a method of the invention) *mutatis mutandis.* "Oral care composition" for the purposes of the present invention means a paste, powder, liquid, gum, serum or other preparation for cleaning the teeth or other surfaces in the oral cavity. "Toothpaste" for the purpose of the present invention means a paste or gel dentifrice for use with a toothbrush.

"Mouth wash" for the purpose of the present invention means liquid dentifrice for use in rinsing the mouth. Especially preferred are mouth washes suitable for rinsing the mouth by swishing and/or gargling for about half a minute before expectorating.

By a 'Haircare Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar.

By A topical composition' or a 'skin care composition' as used herein, is meant to include a composition for topical application to the skin of mammals, especially humans. Such a composition could be of the leave-on or of the wash-off/ rinse-off type. By a leave-on composition is meant a composition that is applied to the desired skin surface and left on for example from one minute to 24 hours, after which it may be wiped or rinsed off with water, usually during the regular course of personal washing. By a wash-off/ rinse off composition is meant a composition that is applied to the desired skin surface for a shorter period, example, of the order of seconds or minutes and usually contains sufficient surfactants that aids in cleaning the surface which may be rinsed off with copious amounts of water. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. "Skin" as used herein is meant to include skin on any part of the body e.g., neck, chest, back, arms, underarms, hands, legs, buttocks and scalp. When the product is used for the underarms it is usually called a deodourant product or a deo product. A class of deodourant product is the so called anti-perspirant (AP) product which contains an AP active which when applied to the axilla of an individual delivers anti-perspirancy and deodourancy benefits.

### The compositions of the invention

In accordance with a first aspect is disclosed a cosmetic composition comprising piperlongumine and at least one of a sulphur-containing amino acid comprising disulphide functional group or a peptide comprising disulphide functional group.

Piperlongumine (or piplarine, 5,6-dihydro-1-[(2E)-1-oxo-3-(3,4,5-trimethoxyphenyl)-2-propenyl]-2(1 H)-pyridinone) is one of the main active alkaloids in long pepper (*Piper longum*) extract. *Piper longum* is commonly used for the treatment of stomach diseases, headache and toothache. Piperlongumine selectively kills tumorous cells without affecting the normal cells by targeting ubiquitin-proteasome system, which gets upregulated in cancerous conditions. Piperlongumine may be isolated from a variety of Piper species (Piperaceae), including *Piper aborescens*, *Piper tuberculatum* and the roots of Piper longum. In addition to its extraction from the roots of the Piper plant, it can also be produced by organic synthesis.

When the composition of the invention comprises a peptide comprising disulphide functional group, it is preferred that the peptide is a linear disulphide peptide such as Glutathione disulphide or a bicyclic peptide with disulphide bonds, preferably one or more of the ones shown hereinbelow.

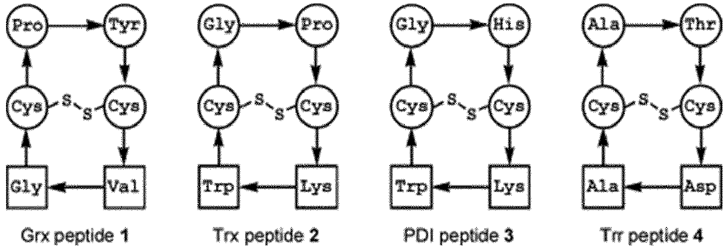

It is clarified that the compositions according to the invention may comprise either a sulphur-containing amino acid comprising disulphide functional group or a peptide comprising disulphide functional group. Alternatively, the compositions of the invention comprise a sulphur-containing amino acid comprising disulphide functional group and a peptide comprising disulphide functional group.

The preferred amino acid is L-cystine. L-cystine is a covalently linked dimeric nonessential amino acid formed by the oxidation of Cysteine. Two molecules of cysteine are joined together by a disulfide bridge to form cystine (Cys-S-S-Cys).

Further preferably the L-cystine is present as a component of a precursor of glutathione which, in addition to the L-cystine, comprises a glutamate source selected from the group consisting of glutamine, glutamine ester, glutamic acid, pyroglutamic acid or its salts, and mixtures thereof. Preferably the precursor of glutathione further comprises glycine.

It is preferred that in the compositions of the invention, ratio of the amount of the piperlongumine to that of the sulphur-containing amino acid or the peptide is from 1:0.01 to 1:100 parts by weight, more preferably from 1:0.1 to 1:20 parts by weight, provided that when said composition comprises the amino acid and the peptide, the combined amount thereof is used for to arrive at the ratio.

The ratios disclosed hereinbefore are generally applicable to e.g., the oral, hair and skin care compositions of the invention. However, the specific amounts of piperlongumine in the compositions could and would vary depending on the nature and purpose of the composition.

Preferably the cosmetic composition of the invention comprises 0.01 to 2 wt% piperlongumine.

It preferred that when the composition of the invention is an oral care composition, the amount of piperlongumine is from 0.01 to 2 wt%.

Alternatively, when the composition of the invention is a hair care composition, the amount of piperlongumine is from 0.01 to 2 wt%.

Further alternatively, when the composition of the invention is a skin care composition, the amount of piperlongumine is from 0.01 to 2 wt%.

As an alternative to piperlongumine which is procured from a natural source or is from synthetic origin, the compositions of the invention could comprise an oil or an extract of, for example, long pepper (*Piper longum*), which in turn contains the requisite amount of piperlongumine. Thus, for example, a cosmetic composition comprising piperlongumine in a cosmetic composition in accordance with the invention could preferably comprise 0.5 to 20 wt% extract of a part of a plant which contains piperlongumine, where said extract comprises piperlongumine. The extract could be aqueous, or that of a non-aqueous solvent or an oil extract. Alternatively, a cosmetic composition in accordance with the invention could preferably comprise 0.5 to 20 wt% oil extracted from a part of a plant which contains piperlongumine, where said oil comprises piperlongumine. The extract or the oil, as the case may be, in turn would contain the amount of piperlongumine desired by the formulation scientist. The wide range applicable to the extract and the oil is partly because the amount of piperlongumine that could possibly be present in the extract or oil is hugely variable and would depend largely on the extraction conditions and the inherent amount of piperlongumine in the part of the plant, which in turn would depend on the source, variety and geographical origin of the plant.

It is preferred that the amino acid is L-cystine, and the amount of L-cystine in said cosmetic composition of the invention is from 0.005 to 5 wt%.

Solubility of L-cystine in water is 0.112 mg/ml at 25°C and L-cystine is more soluble in aqueous solutions with pH less than 2 or pH above 8.

It is further preferred that the L-cystine is present as a component of a precursor of glutathione which, in addition to said cystine, comprises a glutamate source selected from the group consisting of glutamine, glutamine ester, glutamic acid, pyroglutamic acid or its salts, and mixtures thereof.

Further preferably the precursor of glutathione further comprises glycine.

In one embodiment, the precursor of glutathione consists of L-cystine and the glutamate source. Alternatively, the precursor of glutathione consists of L-cystine and glutamate source and glycine.

Preferably the amino acids, glutamine, glutamine ester, glutamic acid, pyroglutamic acid and glycine are present as L stereo isomers, since this is the most abundant and natural isomeric form found in nature. Since the building blocks of naturally-occurring proteins found in human skin, hair and nails are amino acids with the L isomeric form, it is expected that L stereo isomer amino acids contained within personal care products of the present invention can have a greater interaction with these proteins that is intrinsically more biocompatible in nature compared to the D stereo isomeric form. In addition, commercial production and supply of L stereo isomer amino acids is significantly higher compared to the D stereo isomeric form. Finally, L stereo isomer amino acids are also more cost effective to produce, more sustainable, more eco-friendly and available at a lower cost compared to D stereo isomer amino acids. Any of the amino acids included in the present invention may be in the form of a salt, ester, or a salt thereof and the term "cystine," "glutamate source", and "glycine" used in the present specification also encompasses salts, esters, and salts of such esters. The salt, ester, and salt of such ester is not particularly limited as long as it is acceptable for topical application. For example, salts with inorganic acid or organic acid or anionic surfactants can be mentioned. As the inorganic acid, for example, hydrochloric acid, hydrobromic acid, nitric acid, sulfuric acid, phosphoric acid and the like can be mentioned, and as the organic acid C1-C18 linear, branched or cyclic, saturated or unsaturated, unsubstituted or substituted with heteroatoms, for example formic acid, acetic acid, trifluoroacetic acid, propionic acid, lactic acid, tartaric acid, oxalic acid, fumaric acid, succinic acid, maleic acid, citric acid, malonic acid, methanesulfonic acid, stearic acid, oleic acid, 12-hydroxystearic acid, ricinoleic acid, and the like can be mentioned.. As the salt with a base, for example, alkali metal salts such as sodium salt, potassium salt and the like, alkaline earth metal salts such as calcium salt, magnesium salt and the like, and the like can be mentioned. Esters of amino acids are typically C1-C8 esters or salts thereof, or in the alternative C1-C5 esters, or in the alternative C1-C3 esters. Such esters may be straight or branched or cyclic. Especially esters of cystine are beneficial, due to their increased solubility, compared to cystine. Methyl and ethyl esters of cystine or salts thereof are most preferred, due to their efficacy to boost glutathione production and provide antioxidant activity. When salts of esters are used, the same salts are suitable as listed above.

The glutamate source can be present in the form of its functional equivalents - glutamine, glutamic acid and/or pyroglutamic acid and/or their salts may be employed. Pyroglutamic acid (and/or salts thereof) is preferred since it is more stable than glutamine or glutamic acid. In one embodiment, amino acids in GSH precursor are cystine and pyroglutamic acid (and/or salts thereof). In one embodiment, amino acids in GSH precursor are cystine and pyroglutamic acid and glycine (and/or salts thereof).

Preferably the cosmetic composition of the invention comprises 0.01 to 10 wt% of the glutamate source (preferably pyroglutamate) 0.01 to 5 wt%, more preferably 0.05 to 1 wt% and most preferably 0.05 to 0.5 wt%. In one embodiment, glycine source is included in an amount of from 0.01 to 10%, or in the alternative of from to 15 0.01 to 5%, or from 0.01 to 1%, or in the alternative from 0.01 to 0.2%, or in the alternative from 0.01 to 0.1%. In one embodiment, resorcinol is included in an amount of from 0.001% to 3%, or in the alternative of from to 0.01 to 2%, or from 0.05 to 1.5%, or in the alternative from 0.05 to 1.2%.

Preferably the cosmetic composition of the invention is in the form of an oral care composition. Alternatively, it is a skincare composition. Further alternatively it is a hair care composition.

Without wishing to be bound by theory it is believed the mode of action is as follows of the cosmetic composition of the invention is as follows.

The cytotoxic effect of piperlongumine may be attributed to two mechanisms. The first one is ROS (Reactive Oxygen Species) and the other one is disruption of sulphur amino acid homeostasis. Piperlongumine is a known ROS-inducer. However, not all antioxidants can reduce the cytotoxicity of piperlongumine. Vitamin-E is not as effective when it comes to improving the cell viability. This observation suggests that there may be other mechanisms by which piperlongumine's cytotoxicity could be explained.

Glutathione (GSH) is the major native antioxidant in the cells, which requires a sulphur containing amino acid, cysteine. A sulphur-containing amino acid is essential in the synthesis of a wide range of house -keeping proteins. The present inventors believe that ROS induced by piperlongumine can deplete cellular GSH leading to disruption of the homeostasis of sulphur-containing amino acids. So, compounds that could inhibit ROS and provide bioavailable sulphur groups may reduce, and potentially could reduce, the toxicity of piperlongumine. Therefore, the sulphur-containing amino acid comprising disulphide functional group or a peptide comprising disulphide functional group are useful and can widen the therapeutic window of piperlongumine.

### Oral care composition

When the cosmetic composition of the invention is an oral care composition, it includes
a cosmetically acceptable base which will include an abrasive, a thickener, a humectant and orally acceptable surfactant.

Oral care compositions preferably comprise an abrasive. Gels usually contain silica, whereas opaque creams generally contain calcium based abrasives, especially chalk. The oral care composition of the invention could be a toothpaste a mouthwash or any other suitable format including teeth whitening strips.

Preferably the toothpaste composition will comprise 5 to 60 wt% calcium based abrasive, more prerferably 30 to 60 wt% and furthermore preferably 35 to 55 wt%.

A preferred type of abrasive is fine ground natural chalk (FGNC), which is a form of chalk. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling. FGNC may be used as the sole calcium based abrasive. However, FGNC may also be used with the other calcium based abrasives for some balance of abrasion. Usually the particle size of chalk is from 1 to 60 µm, and preferred sizes range from 1 to 15 µm.

Other preferred calcium-based abrasives include dicalcium phosphate (DCP), calcium pyrophosphate and precipitated calcium carbonate (PCC), which preferably are included at 25 to 55 wt%, more preferably 35 to 50 wt% by weight of the composition.

When a combination of calcium based abrasives is desired, it is preferred that FGNC is 35 to 100%, more preferably 75 to 100% and especially from 95 to 100% of the total amount of Calcium based abrasives. In such cases, the balance, most preferably, is PCC.

Other abrasives may also be used depending upon the intended degree of abrasion. These include synthetic abrasive polishing agents such as amorphous precipitated silica and silica gels. Other abrasive agents include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

It is preferred that oral care composition of the invention comprises a thickener. Thickeners that may be used in this invention include, sodium carboxymethyl cellulose (SCMC), hydroxyl ethyl cellulose, methyl cellulose, ethyl cellulose, gum tragacanth, gum arabic, gum karaya, xanthan gum, sodium alginate, carrageenan gum, guar gum, Irish moss, starch, modified starch, silica based thickeners including silica aerogels, magnesium aluminum silicate (e.g., Veegum®), Carbomers (cross-linked acrylates) and mixtures thereof.

Typically, thickening silica, sodium carboxymethyl cellulose and/or a Carbomer is/are preferred thickeners for use in the composition of the invention. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein. In an especially preferred embodiment, the Carbomer is Synthalen® PNC, Synthalen® KP or a mixture thereof. It has been described as a high molecular weight and cross- linked polyacrylic acid and identified via CAS number 9063-87-0. These types of materials are available commercially from suppliers like Sigma. In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickening silica is especially preferred for use in gel toothpastes.

Gel toothpastes generally contain upto 8.5 wt% thickening silica whereas opaque toothpastes typically contain 3 to 4 wt% thickening silica. When present, preferred thickening silicas include AEROSIL® T series from Degussa or the CAB-O-SIL® series from Cabot Corporation, silica gels such as the SYLODENT® or SYLOX® series from W. R.Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL, MFIL-P (From Madhu Silica, India), SIDENT® 22 S and AEROSIL® 200 (Ex. Evonik Industries), SYLODENT® and PERKASIL® thickening silicas from WR Grace & Company and Tixosil® 43 and 331 from Rhodia synthetic finely divided pyrogenic silica such as those sold under the trademarks SYLOID® 244, SYLOID® 266 and AEROSIL® D-200. Thickener, when present, preferably makes up from 0.01 to about 10%, more preferably from 0.1 to 9%, and most preferably, from 1.5 to 8% by weight of the composition.

Preferably the oral care composition of the present invention comprises a humectant. Preferred humectants are glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol and sorbitol are the more preferred humectants.

Preferably the oral care composition comprises 10 to 90 wt% humectant, more preferably 25 to 80 wt% and most preferably 45 to 70 wt%.

Preferably, the oral care composition comprises a surfactant. Preferably the composition comprises at least 0.01 wt% surfactant, more preferably at least 0.1 wt% and most preferably from 0.5 to wt7%. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. More preferably the surfactant comprises or is an anionic surfactant. The preferred anionic surfactants are sodium lauryl sulphate and/or sodium dodecylbenzene sulfonate. Most preferably the surfactant is sodium lauryl sulphate. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Most preferred surfactants are an alkali metal alkyl sulphate or a betaine.

The oral care composition preferably comprises 5 to 95 wt% water, more preferably 10 to 75 wt%, and especially at from 10 to 60 wt%.

When the oral care composition of this invention is a toothpaste or gel, the viscosity is preferably from 30,000 to 180,000 centipoises, more preferably, 60,000 to 170,000 centipoises, and most preferably 65,000 to 165,000 centipoises.

The oral care composition of the present invention may contain a variety of other ingredients which are common in the art to enhance physical properties and performance. These ingredients include antimicrobial agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20 wt% of the composition.

### Hair Care

The cosmetic composition of the invention could alternatively be a haircare composition. It is especially useful for preventing or alleviating the symptoms of dandruff. One medium through which this may be delivered is that of a shampoo. The composition of the invention especially shampoos are formulated with an anionic surfactant e.g. an alkyl sulphate and/or ethoxylated alkyl sulfate surfactant. Preferably the composition comprises 1 to 20 wt%, more preferably 2 to 16 wt% anionic surfactants. Preferred alkyl sulfates are C8 to 18 alkyl sulfates, more preferably C12 to 18 alkyl sulfates, preferably in the form of a salt with a solubilising cation such as sodium, potassium, ammonium or substituted ammonium.

Preferred alkyl ether sulfates are those having the formula: RO(CH2CH2O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES). SLES having an average degree of ethoxylation of from 0.5 to 3, preferably 1 to 3 is especially preferred. The haircare composition of the invention preferably also comprises an amphoteric surfactant, preferably a betaine and more preferably an alkyl amidopropyl betaine surfactant for example cocamidopropyl betaine. It is preferred that the haircare composition comprises 0.1 to 10 wt%, preferably 0.5 to 8 wt%, more preferably from 1 to 5 wt% betaine surfactant.

To enhance deposition of actives by use of haircare compositions of the invention as a delivery vehicle, especially shampoos; cationic polymers are generally included. In the present invention too, it is preferred that the composition additionally comprises 0.01 to 2.0 wt% of a cationic polymer. Preferably the polymer is guar hydroxypropyl trimonium chloride. Guar predominantly contains galactomannan polymer chains. This polymer can have a wide range of molecular weight and degree of cationic substitution depending on how much of the guar has been hydrolysed and cationised. Compositions of the invention preferably comprise 0.04 to 0.5 wt%, more preferably 0.08 to 0.25 wt% guar. When conditioning benefits are to be delivered through the composition of the invention, the composition is called a hair conditioner. Typically, the most popular conditioning agents used in hair care compositions are water-insoluble oily materials such as mineral oils, naturally occurring oils such as triglycerides and silicone polymers.

Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Coming 9040 and 9041). Amounts of the silicone in compositions where present may range from about 0.1 to 10 wt%, preferably from 0.1 to 8 wt%, more preferably from 0.3 to 5 wt%.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in the conditioners include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad® 2HT/75 from Akzo Nobel), cocotrimethylammonium chloride, PEG-2- oleammonium chloride and the corresponding hydroxides thereof. Further suitable cationic surfactants include those materials having the CTFA designations Quatemium®-5, Quaternium®-31 and Quatemium-18. Mixtures of any of the foregoing materials may also be suitable. A particularly useful cationic surfactant for use in conditioners according to the invention is cetyltrimethylammonium chloride, available commercially, for example as GENAMIN® CTAC, ex Hoechst Celanese. Another particularly useful cationic surfactant for use in conditioners according to the invention is behenyltrimethylammonium chloride, available commercially, for example as GENAMIN® KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners, the level of cationic surfactant will generally range from 0.1 to 5 wt%, preferably 0.5 to 2.5 wt% by weight of the composition.

Hair conditioning compositions of the invention preferably may further comprise a fatty alcohol. The combined use of fatty alcohols and cationic surfactants in conditioning compositions is believed to be advantageous especially because this leads to the formation of a lamellar phase in which the cationic surfactant is dispersed.

Representative fatty alcohols comprise from 8 to 22 carbon atoms, more preferably 16 to 22. Fatty alcohols are typically compounds that contain straight chain alkyl groups. Examples of suitable fatty alcohols include cetyl alcohol, stearyl alcohol and mixtures thereof. The use of these materials is also advantageous in that they contribute to the overall conditioning properties of compositions of the invention. The level of fatty alcohol in conditioners of the invention will generally range from 0.5 to 10 wt%, preferably from 0.1 to 8 wt%, more preferably from 0.2 to 7 wt%. The weight ratio of cationic surfactant to fatty alcohol is suitably from 1:1 to 1:10, more preferably from 1:1.5 to 1:8, optimally from 1:2 to 1:5.

Hair care compositions, whether shampoos or conditioners, usually comprise an anti-dandruff agent. The most preferred anti-dandruff agent is a zinc-based anti-dandruff agent, preferably zinc pyrithione. It belongs to the class of insoluble metal pyrithiones.

It is preferred that the composition of the invention comprises 0.01 to 1.5% zinc pyrithione, more preferably 0.05 to 1.5 wt%.

The composition as per the invention especially anti-dandruff shampoo preferably also comprises a zinc-based compound. The presence of additional zinc compound can improve the antidandruff efficacy of the zinc-based antidandruff agent. Suitable zinc-based compounds are zinc oxide, zinc citrate, zinc malonate, zinc carbonate or a combination thereof. It is preferred that the composition comprises 0.1 to 3 wt%, more preferably 0.1 to 1.5 wt% zinc-based compound.

### Skin Care

The composition of the invention may alternatively be formulated for use as a skin care composition.

Such compositions usually comprise a cosmetically acceptable base which may be liquid or solid. Typically, the base may constitute 10 to 90 wt%, more preferably 20 to 95 wt% and most preferably 40 to 85 wt% of the composition including. It is particularly preferred that the cosmetically acceptable carrier includes water, more preferably 30 to 90 wt%, still more preferably from 30 to 85 wt% and most preferably 30 to 80 wt%. In addition to water, the carrier may comprise or even consist of silicones, polyhydric alcohols, hydrocarbons, triglycerides and thickening powders.

The skin care composition of the invention may be in any form including toners, lotions, creams, mousses, scrub, serum or a gel that is suitable for topical application to the skin. The composition can be either a leave-on product such as skin lotions, creams, antiperspirants, deodorants, lipsticks, foundations, mascara, sunless tanners and sunscreen lotions, or a rinse-off product such as shower gels and toilet bars. It is preferred that the composition is a leave-on skin lotion or a cream, especially for cosmetic treatment of acne.

The skincare composition of the invention preferably comprises an emollient oil that acts as a cosolvent. Suitable emollient oils include esters of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and mixtures thereof. Preferably the emollient oil is caprylic/capric triglyceride.

The skin care composition of the invention may also comprise sunscreen agents such as inorganic sunscreens, for example, zinc oxide, titanium dioxide, iron oxide and silica such as fumed silica. Preferably the amount of such sunscreen agents is 0.1 to 5 wt%. The composition of the invention may also comprise a UV-A sunscreen agent selected from the group consisting of a dibenzoylmethane derivative, a triazine derivative, a benzophenone derivative and mixtures thereof. In a preferred embodiment, the UV-A sunscreen agent comprises or is a dibenzoylmethane derivative, for example, butyl methoxydibenzoylmethane (sold under the trade name Parsol® 1789). It is preferred that the skin care composition of the invention comprises 0.1 to 15 wt% UV-A sunscreen agent, more preferably 0.1 to 10 wt%, most preferably from 1 to 5 wt%.

In addition, the composition of the invention may also comprise a UV-B sunscreen agent. Suitable UV-B sunscreen agents include benzophenone, an anthranilate, a salicylate, a cinnamate, a camphor, benzylidene malonate, a triazone, and derivatives thereof. In a preferred embodiment, the UV-B sunscreen agent comprises or is a cinnamate derivative, for example, ethylhexyl methoxycinnamate (sold under the trade name Parsol® MCX). It is preferred that the skin care composition of the invention comprises 0.1 to 20 wt%, more preferably 0.5 to 18 wt% UV-B sunscreen agent.

A skin lightening agent may also be included. Suitable skin lightening agents include niacin, niacinamide, kojic acid, arbutin, tranexamic acid, placental extract, ascorbic acid and its derivatives (e.g. magnesium ascorbyl phosphate, sodium ascorbyl phosphate, ascorbyl glucoside, and ascorbyl tetraisopalmitates), aloe extract, ammonium lactate, azelaic acid, citrate esters, ellagic acid, glycolic acid, green tea extract, hydroquinone, lemon extract, linoleic acid, vitamins like vitamin B6, vitamin B12, vitamin C, vitamin A, a dicarboxylic acid, resorcinol derivatives, hydroxycarboxylic acid like lactic acid and their salts (e.g. sodium lactate) or a mixture thereof. It is preferred that the composition of the invention comprises 0.1 to 10 wt%, more preferably 0.2 to 5 wt%, most preferably from 0.3 to 3 wt% skin lightening agent.

The composition may also comprise other ingredients which are common in the art to enhance physical properties and performance. Suitable ingredients include but are not limited to humectants, thickeners, opacifiers, binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti- aging agents, anti-acne agents, anti-microbial agents and antioxidants.

### Method of the Invention

In accordance with another aspect of the invention is disclosed a method of widening the therapeutic window of piperlongumine for its use in a cosmetic composition for cosmetic treatment of an inflammatory condition including acne, dandruff and gingivitis, comprising including in said composition at least one of a sulphur-containing amino acid comprising disulphide functional group or a peptide comprising disulphide functional group.

As per the method of the invention, it is preferred that the inflammatory conditions is caused by lipopolysaccharides of Gram Negative bacteria. Further preferably the Gram-Negative bacteria is the bacterium *E*. *coli.*

It is preferred that the inflammatory condition is at least one of acne, dandruff or gingivitis. Accordingly, the method in accordance with this invention is skin care, hair care or oral care method respectively.

In accordance with one aspect, the method of this invention is a therapeutic method. Alternatively, and more preferably, the method is a non-therapeutic method. Non-therapeutic preferably means a cosmetic method for the cosmetic treatment of acne, dandruff and gingivitis, which is clearly distinct from therapeutic or medical treatment.

Preferably the method in accordance with this invention is demonstrated or exemplified by performing a THP-1 invitro assay on Human immune cell line. In this assay, the concentration of IL-6 (Interleukin-6) and the % cell viability are measured. Widening of therapeutic window is indicated by the concentration of Interleukin-6 (IL-6) as measured on the Human Immune cell Line using the THP-1 invitro assay in pg/ml and the % cell viability of THP-1 cells.

### Use in accordance with the invention

In accordance with yet another aspect is disclosed the use of a sulphur-containing amino acid comprising disulphide functional group or a peptide comprising disulphide functional group in a cosmetic composition comprising piperlongumine, to widen the therapeutic window of piperlongumine in said cosmetic composition for cosmetic treatment of an inflammatory condition including acne, dandruff and gingivitis.

Preferably the use is for non-therapeutic purpose. Alternatively it is for therapeutic purpose.

In accordance with another aspect is disclosed a topical composition comprising piperlongumine and a sulphur-containing amino acid or protein comprising disulphide functional group for use in the treatment of inflammatory conditions including acne, dandruff and gingivitis.

The invention will now be described with reference to the following non-limiting examples.

### Examples

### Example 1

### Human immune cell line THP-1 invitro assay

The following procedure was used to determine the anti-inflammation efficacy:
THP1-XBlue™ (Cat No: thpx-sp, InvivoGen) cells were cultured as suspense in RPMI 1640 medium supplemented with 10% FBS, penicillin (10 U/mL) - streptomycin (10 µg/ML). Cells were differentiated in 24-well plates at the density of 5 x 10⁵ cells / well with 100 nM PMA for 72 hours. Cells were then co-treated with *E.coli* lipopolysaccharides (LPS) and single concentration or a combination of piperlongumine and L-cystine at different doses. After 24 hours, the supernatants were collected for measurement of interleukin (IL)-6 as a pro-inflammatory biomarker using enzyme-linked immunosorbent assay (ELISA). IL-6 is a cytokine, or cell-signaling protein, encoded in humans by the IL- 6 gene which is both pro-inflammatory and anti-inflammatory and which stimulates the immune response to trauma leading to inflammation. The results in terms of concentration of IL-6 in pg/mL and cell viability are given in Table 1:

**Table 1**

| **Example** | **Composition** | **Concentration of IL-6 (pg/mL)** | **Std. dev of IL-6** | **Cell viability (%)** | **Std. dev of cell viability** |
|---|---|---|---|---|---|
| 1 | Control | 98 | 13.07 | 100 | 9.14 |
| 2 | LPS | 2252 | 256.51 | 91 | 6.88 |
| 3 | L-cystine 100 µM | 1896 | 284.93 | 90 | 5.57 |
| 4 | Piperlongumine 10 µM | 117 | 21.54 | 74 | 3.34 |
| 5 | Piperlongumine 5 µM | 103 | 21.60 | 100 | 4.15 |
| 6 | Piperlongumine 10 µM + L-cystine 100 µM | 128 | 47.94 | 94 | 3.89 |
| 7 | Piperlongumine 5 µM + L-cystine 100 µM | 116 | 42.52 | 111 | 5.22 |

The basal level concentration of IL-6 was 98 pg/ml. In the Example 2, LPS increased the production of pro-inflammatory cytokine IL-6 to 2252 pg/mL. Example 4 and Example 5 were different doses of piperlongumine which suppressed LPS-induced IL-6 to 117 pg/mL and 103 pg/mL, respectively. However, the cell viability dropped to 74%. Examples 6 and 7 were the combination of L-cystine with piperlongumine at differring doses. The data in Table 1 indicates that combination of 10 µM L-cystine rescued 10 µM piplerongumine-induced cell loss from 74% to 94%. In addition, L-cystine could further increase the cell viability of 5 µM piperlongumine to 111%. The IL-6 production in Example 6 and 7 was 128 pg/mL and 116 pg/mL respectively.

Taken together, the data in Table 1 indicates that a combination of L-cystine (a sulphur-containing amino acid comprising disulphide functional group) with piperlongumine protects THP-1 cells from cytotoxicity induced by piperlongumine. Meanwhile, the anti-inflammatory effects of piperlongumine is not compromised by L-cystine.

Table 2 summarizes the combination results of piperlongumine with vitamin E on the concentration of IL-6 and the effect on cell viability:

**Table 2**

| **Examples** | **Composition** | **Concentration of IL-6 (pg/mL)** | **Std. dev of IL-6** | **Cell viability (%)** | **Std. dev of cell viability** |
|---|---|---|---|---|---|
| 8 | Control | 122 | 36.03 | 100 | 2.40 |
| 9 | LPS | 3953 | 524.34 | 86 | 4.30 |
| 10 | Piperlongumine 10 µM | 91 | 5 | 39 | 3.44 |
| 11 | Vitamin E 100 µM | 2998 | 165 | 73 | 0.95 |
| 12 | Vitamin E 100 µM + Piperlongumine 10 µM | 70 | 12 | 48 | 7.44 |

Piperlongumine 10 µM in Example 10 reduced the IL-6 concentration from 3953 pg/mL (Example 9) to 91 pg/mL, however the cell viability declined to 39%. In Example 12 the cell viability was 48%, which was not a significant increase over Example 10.

Therefore, the data in Table 2 indicates that vitamin-E cannot rescue THP-1 cells from the cytotoxicity induced by piperlongumine.

### Examples 13 to 18

### Human oral keratinocytes (HOK) in vitro assay

HOKs isolated from normal human oral mucosa (Cat. 2610, Sciencell, CA, USA) were cultured according to the manufacturer's instructions. Cells were cultured with 500 mL complete oral keratinocyte medium by adding 10 mL of keratinocyte growth supplement and 5 mL of penicillin/streptomycin solution (Cat. 2101, Sciencell). HOKs (Passage 4-6) were seeded in 96 well-plates at the density of 2 x 10⁴ cells / well overnight. On the experimental day the cells were treated with heat killed *P.gingivalis* and single concentration or a combination of piperlongumine and L-cystine at different doses. After 24 hours, cell viability was measured with cell counting kit-8. The results in terms of cell viability in HOKs are shown in Table 3:

**Table 3**

| **Example** | **Composition** | **Cell viability (%)** | **Std. dev of cell viability** |
|---|---|---|---|
| 13 | Control | 100 | 4.664054826 |
| 14 | Piperlongumine 15 µM | 63.37587914 | 4.147406274 |
| 15 | L-cystine 50 µM | 102.0643397 | 4.655773359 |
| 16 | L-cystine 100 µM | 109.2862725 | 4.670233588 |
| 17 | Piperlongumine 15 µM + L-cystine 50 µMµM | 101.0506208 | 5.517005981 |
| 18 | Piperlongumine 15 µM + L-cystine 100 µM | 91.0067726 | 4.154020043 |

In Example 14 the HOKs were exposed to 15 µM piperlongumine, which reduced the cell viability to 63.4%. Example 15 and Example 16 were treatments of L-cystine at different doses and L-cystine alone did not impair the cell viability. Example 17 and Example 18 were the combination of piperlongumine and L-cystine. The viability in Example 17 and 18 recovered to 101% and 91%, respectively.

In conclusion, the data in Table 3 indicates that a combination of L-cystine and piperlongumine can protect HOK cells from cytotoxicity induced by piperlongumine.

## Claims

1. A cosmetic composition comprising piperlongumine and at least one of a sulphur-containing amino acid comprising disulphide functional group or a peptide comprising disulphide functional group.

2. A cosmetic composition as claimed in claim 1 wherein said amino acid is L-cystine.

3. A cosmetic composition as claimed in claim 2 wherein said L-cystine is present as a component of a precursor of glutathione which, in addition to said L-cystine, comprises a glutamate source selected from the group consisting of glutamine, glutamine ester, glutamic acid, pyroglutamic acid or its salts, and mixtures thereof.

4. A cosmetic composition as claimed in claim 3 wherein said precursor of glutathione further comprises glycine.

5. A cosmetic composition as claimed in any of claims 1 to 4 wherein, in said composition, ratio of the amount of said piperlongumine to that of said sulphur-containing amino acid or said peptide is from 1:0.01 to 1:100 parts by weight, provided that when said composition comprises said amino acid and said peptide, the combined amount thereof is used for to arrive at said ratio.

6. A cosmetic composition as claimed in any of claims 1 to 5 wherein said composition comprises 0.01 to 2wt% piperlongumine.

7. A cosmetic composition as claimed in claim 6 wherein said composition comprises 0.005 to 5 wt% L-cystine.

8. A cosmetic composition as claimed in any of claims 1 to 7 wherein said composition an oil or an extract of long pepper (*Piper longum*), which in turn contains the requisite amount of piperlongumine.

9. A composition as claimed in any of claims 1 to 8 wherein said composition is an oral care, a skincare, or a hair care composition.

10. A method of widening the therapeutic window of piperlongumine for its use in a cosmetic composition for cosmetic treatment of an inflammatory condition including acne, dandruff and gingivitis, comprising including in said composition at least one of a sulphur-containing amino acid comprising disulphide functional group or a peptide comprising disulphide functional group.

11. A method as claimed in claim 10 wherein said inflammatory condition is caused by lipopolysaccharides of Gram Negative bacteria.

12. A method as claimed in claim 11 wherein one of said Gram Negative bacteria is the bacterium *E*. *coli.*

13. A method as claimed in any of claims 10 to 12 wherein said widening of therapeutic window is indicated by the concentration of Interleukin-6 (IL-6) as measured on Human Immune cell Line using the THP-1 invitro assay in pg/ml and the % cell viability of THP-1 cells.

14. Use of a sulphur-containing amino acid comprising disulphide functional group or a peptide comprising disulphide functional group in a cosmetic composition comprising piperlongumine, to widen the therapeutic window of piperlongumine in said cosmetic composition for cosmetic treatment of an inflammatory condition including acne, dandruff and gingivitis.

15. A topical composition comprising piperlongumine and a sulphur-containing amino acid or protein comprising disulphide functional group for use in the treatment of inflammatory conditions including acne, dandruff and gingivitis.
